# FASCICULE DE BREVET EUROPEEN

(11) **EP 0 957 821 B1**
(45) Date de publication et mention de la délivrance du brevet: **04.06.2003**
(21) Numéro de dépôt: 96927092.5
(22) Date de dépôt: 26.07.1996
(51) Int. Cl.: A61F 2/06

(54) **ENDOPROTHESE INTRALUMINALE DESTINEE EN PARTICULIER A L'ANGIOPLASTIE**
INTRALUMINALE ENDOPROTHESE INSBESONDERE FÜR DIE ANGIOPLASTIE
INTRALUMINAL ENDOPROSTHESIS, PARTICULARLY FOR ANGIOPLASTY

(30) Priorité: 24.08.1995 FR 9510061
(43) Date de publication de la demande: 24.11.1999
(73) Titulaire: Rieu, Régis, 13008 Marseille (FR); Bergeron, Patrice, 13009 Marseille (FR)
(72) Inventeur: Rieu, Régis, 13008 Marseille (FR); Bergeron, Patrice, 13009 Marseille (FR)
(74) Mandataire: Breese, Pierre
(86) Numéro de dépôt international: FR9601185
(87) Numéro de publication internationale: WO97007752

(56) Documents cités:
- EP-A- 0 566 807
- EP-A- 0 622 059
- DE-A- 4 109 015
- FR-A- 2 678 508

## Description

La présente invention concerne une endoprothèse intraluminale destinée en particulier à l'angioplastie.

De telles prothèses sont connues, par exemple, sous le nom d'endoprothèses de PALMAZ (marque déposée) commercialisées par la société JOHNSON & JOHNSON et sont présentées dans l'article « introduction to intravasculare stents » de Richard A. SCHATZ paru dans « CARDIOLOGY CLINICS- Vol. 6 N° 3, August 1988 ». Ces endoprothèses sont constitués par un matériau permettant une expansion radiale avec une sonde à ballonnet. Leur enveloppe présente des découpes, par exemple en losange, permettant cette déformation expansive et augmentant la surface artérielle libre de métal après implantation. Elles sont destinées à maintenir la paroi vasculaire.

De telles endoprothèses offrent satisfaction dans la plupart des cas. Toutefois, lorsqu'elle est implantée à proximité d'une bifurcation, il arrive que son extrémité dépasse dans le canal médullaire de la branche saine, et perturbe ainsi le flux sanguin.

Il est toutefois apparu que les endoprothèses implantées à proximité d'une bifurcation connaissent une endothélialisation irrégulière et souvent incomplète, contrairement aux implantations sur des vaisseaux rectilignes tels que des vaisseaux fémoraux.

Les chirurgiens considèrent qu'une endoprothèse ne doit pas prolaber, et l'implante en conséquence légèrement en retrait de la bifurcation, ce qui empêche de recouvrir la totalité de la zone malade, qui pourtant s'étend souvent sur une partie de l'autre branche.

Pour remédier à cet inconvénient, on a proposé dans un article ayant pour titre « aortic bifurcation stenosis : treatment with intravascular stents » paru dans « RADIOLOGY-JVIR, Vol. 2, N°3, August 1991, pp319-323, d'implanter des endoprothèses dans les deux branches sténosées, et de faire déboucher les extrémités des deux endoprothèses parallèlement dans la branche commune des vaisseaux. Cette solution permet certes de recouvrir la totalité des zones atteintes, et de maintenir un flux sanguin sensiblement laminaire, mais présente l'inconvénient de réduire sensiblement la section utile de la branche commune, et ne peut être utilisée que pour des vaisseaux suffisamment larges.

On a également proposé dans le brevet français FR2678508 une endoprothèse comprenant une armature allongée épousant la paroi interne du vaisseau à renforcer. En vue de permettre de réaliser des jonctions continues notamment au niveau de bifurcations, deux dispositifs au moins sont prévus comportant des spires auto-verrouillantes permettant de réaliser des connections continues.

Un autre brevet français publié sous le numéro FR2671280 décrit une endoprothèse constituée de plusieurs unités modulaires comportant chacune des axes longitudinaux souples et élastiques, reliés entre eux par des brins en forme de « V » imbriqués les uns dans les autres prenant. un aspect en « arête de poisson » ; 2, 3 ou plusieurs unités réunis longitudinalement forment un cylindre élastique. Ce dispositif peut être introduit dans une artère par un cathéther et un dispositif d'application qui permet d'étendre le cylindre ajouré pour faire diminuer son diamètre puis de le laisser reprendre sa forme initiale.

De telles endoprothèses ne résolvent pas le problème de l'invention et se traduisent en outre par une fabrication difficile et coûteuse.

L'invention a pour objet de remédier à ces différents inconvénients en proposant une endoprothèse améliorée permettant d'appareiller les vaisseaux au niveau d'une bifurcation en préservant un comportement hydrodynamique satisfaisant.

A cet effet, l'invention concerne particulièrement une endoprothèse vasculaire selon la revendication 1. Cette endoprothèse présente la forme générale d'un cylindre ajouré expansible et la longueur des génératrices de l'endoprothèse expansée, mesurée par rapport à un plan transversal déterminé, n'est pas constante.

L'une des extrémités au moins du cylindre ajouré est coupée sensiblement en biseau.

L'une des extrémités frontales au moins est délimitée par un plan formant avec l'axe longitudinal un angle inférieur à 90°, préférentiellement un angle compris entre 30° et 60°.

Selon une deuxième variante de réalisation, l'une des extrémités frontales au moins présente la forme de l'intersection de deux cylindres perpendiculaires.

Selon une troisième variante de réalisation l'une des extrémités frontale au moins présente, vue de face, une section en « V ». Ce mode de réalisation permet l'implantation dans une branche commune et le raccordement de deux endoprothèses selon la première ou deuxième variante, implantées dans les branches secondaires.

Avantageusement, l'endoprothèse selon l'invention comporte des points de marquage radio-opaques formant un trièdre pour le repérage de l'orientation lors de l'implatation.

L'invention sera mieux comprise à la lecture de la description d'un exemple non limitatif de réalisation qui suit, faisant référence aux dessins annexés où :
- la figure 1 représente une vue de face d'une endoprothèse selon l'invention ;
- la figure 2 représente une vue schématique en coupe d'un exemple d'implantation au niveau d'une bifurcation vasculaire.

L'endoprothèse est formée par un cylindre ajouré en acier inoxydable d'un diamètre non expansé de 3,1 millimètres, et d'un diamètre expansé de 8 millimètres. Il présente un réseau de lumières (1) polygonales et de noeuds (2). L'une des extrémités frontales (5) se termine selon un plan transversal (3) perpendiculaire à l'axe longitudinal (4).

L'autre extrémité (6) se termine en biseau. Elle est délimitée par un plan (7) formant avec l'axe longitudinal (4) un angle d'environ 30°. La longueur, mesurée à partir d'un plan transversal déterminé (9), par exemple le plan médian, des génératrices (8) de l'enveloppe du corps tubulaire ajouré, varie entre une valeur minimale Lmin et une valeur maximale Lmax.

L'extrémité en biseau (6) est découpée au niveau des noeuds (10).

Trois points de marquage radio-opaques (11 à 13) définissent un trièdre permettant le repérage de l'orientation lors de l'implantation, et son repositionnement avant l'expansion.

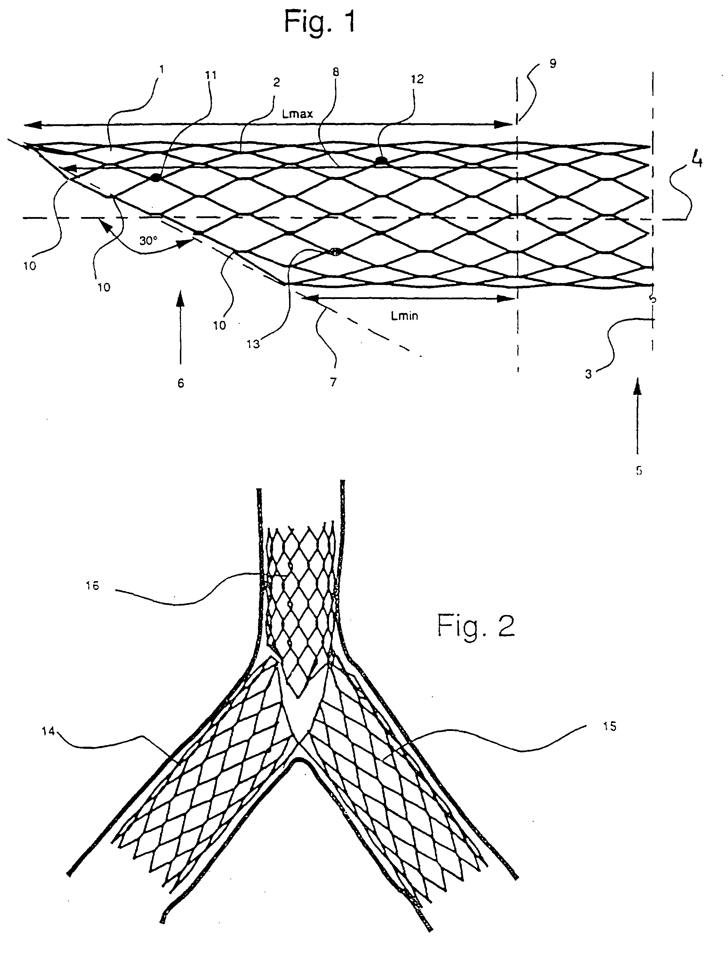

La figure 2 représente une vue schématique en coupe d'un exemple d'implantation au niveau d'une bifurcation vasculaire. Dans cet exemple, on utilise deux endoprothèses (14, 15) d'un premier type présentant, en vue de face, une extrémité coupée selon un plan formant un angle d'environ 50 degrés par rapport à l'axe longitudinal, et une endoprothèse d'un deuxième type (16) présentant une extrémité coupée selon deux plans et présentant, vue de face, une forme de « V ».

L'invention est décrite dans ce qui précède à titre d'exemple non limitatif. Il est bien entendu que l'Homme de Métier sera à même de réaliser différentes variantes sans pour autant sortir du cadre de l'invention telle que revendiquée.

## Revendications

1. Endoprothèse vasculaire (14, 15, 16) pour maintenir la paroi vasculaire d'une bifurcation vasculaire, présentant la forme générale d'un cylindre expansible radialement, **caractérisée en ce que** l'extrémité (6) de l'endoprothèse destinée à s'engager dans la bifurcation est coupée sensiblement en biais, afin que cette extrémité (6) ne soit pas prolabée.

2. Endoprothèse vasculaire (14, 15, 16) selon la revendication 1 **caractérisée en ce que** l'extrémité (6) coupée en biais est délimitée par un plan formant avec l'axe longitudinal (4) un angle inférieur à 90°

3. Endoprothèse vasculaire (14, 15, 16) selon la revendication 2 **caractérisée en ce que** l'extrémité (16) coupée en bais est délimitée par un plan formant avec l'axe longitudinal (4) un angle compris entre 30° et 60°.

4. Endoprothèse vasculaire (14, 15, 16) selon la revendication 1 **caractérisée en ce que** l'une des extrémités frontales au moins présente la forme de l'intersection de deux cylindres perpendiculaires.

5. Endoprothèse vasculaire (16) selon la revendication 1 **caractérisée en ce que** l'extrémité de l'endoprothèse destinée à s'engager dans la bifurcation présente, vue de face, une section en « V ».

6. Endoprothèse vasculaire (14, 15, 16) selon l'une quelconque des revendications précédentes **caractérisée en ce qu'**elle comporte des points de marquage (11, 12, 13) radio-opaques formant un trièdre pour le repérage de l'orientation lors de l'implantation.

## Patentansprüche

1. Gefäßendoprothese (14, 15, 16) um die Gefäßwand einer Gefäßbifurkation zu halten, welche eine allgemeine radial ausdehnbare zylindrische Form aufweist, **dadurch gekennzeichnet, dass** das Ende (6) der Endoprothese, welches dazu bestimmt ist, sich in die Bifurkation einzufügen, wesentlich schräg geschnitten ist, damit dieses Ende (6) nicht vorfällt.

2. Gefäßendoprothese (14, 15, 16) nach Anspruch 1, **dadurch gekennzeichnet, dass** das schräg geschnittene Ende (6) durch eine Ebene abgegrenzt ist, welche mit der Längsachse (4) einen Winkel kleiner als 90° bildet.

3. Gefäßendoprothese (14, 15, 16) nach Anspruch 2, **dadurch gekennzeichnet, dass** das schräg geschnittene Ende (6) durch eine Ebene abgegrenzt ist, welche mit der Längsachse (4) einen Winkel zwischen 30° und 60° bildet.

4. Gefäßendoprothese (14, 15, 16) nach Anspruch 1, **dadurch gekennzeichnet, dass** mindestens eins der Stirnenden die Form der Kreuzung von zwei zueinander senkrechten Zylindern aufweist.

5. Gefäßendoprothese (16) nach Anspruch 1, **dadurch gekennzeichnet, dass** das Ende der Endoprothese, welches dazu bestimmt ist, sich in die Bifurkation einzufügen, von vorne gesehen einen V-Schnitt aufweist.

6. Gefäßendoprothese (14, 15, 16) nach einem der vorangehenden Ansprüche, **dadurch gekennzeichnet, dass** sie für Röntgenstrahlen undurchlässige Markierungspunkte (11, 12, 13) aufweist, welche zum Orten der Orientierung bei der Einpflanzung ein Trieder bilden.

## Claims

1. Vascular endoprosthesis (14, 15, 16) to hold the vascular wall of a vascular bifurcation, having an overall radially extensible cylindrical shape, **characterised in that** the end (6) of the endoprosthesis intended to be inserted into the bifurcation is cut roughly at an angle, so that said end (6) is not prolapsed.

2. Vascular endoprosthesis (14, 15, 16) according to claim 1 **characterised in that** the end (6) cut at an angle is delimited by a plane forming with the longitudinal axis (4) an angle less than 90°.

3. Vascular endoprosthesis (14, 15, 16) according to claim 2 **characterised in that** the end (6) cut at an angle is delimited by a plane forming with the longitudinal axis (4) an angle between 30° and 60°.

4. Vascular endoprosthesis (14, 15, 16) according to claim 1 **characterised in that** one of the front ends at least has the shape of the intersection of two perpendicular cylinders.

5. Vascular endoprosthesis (14, 15, 16) according to claim 1 **characterised in that** the end of the endoprosthesis intended to be inserted into the bifurcation has, viewed from the front, a V-shaped cross-section.

6. Vascular endoprosthesis (14, 15, 16) according to any of the above claims **characterised in that** it comprises radio-opaque marking points (11, 12, 13) forming a trihedron for indicating the orientation during fitting.
